Europäisches Patentamt

European Patent Office

Office européen des brevets

(11) Publication number: **0 109 060**
**B1**

# EUROPEAN PATENT SPECIFICATION

(45) Date of publication of patent specification: **11.03.87**

(21) Application number: **83111241.2**

(22) Date of filing: **10.11.83**

(51) Int. Cl.⁴: **C 07 C 11/06, C 07 C 4/10, B 01 J 29/28, B 01 J 29/04**

(54) **Process for the conversion of linear butenes to propylene.**

(30) Priority: **10.11.82 IT 2415382**
**24.12.82 IT 2497982**
**14.03.83 IT 2005383**
**03.05.83 IT 2089683**

(43) Date of publication of application:
**23.05.84 Bulletin 84/21**

(45) Publication of the grant of the patent:
**11.03.87 Bulletin 87/11**

(84) Designated Contracting States:
**DE FR GB IT**

(56) References cited:
**EP-A-0 037 671**
**US-A-4 150 062**

(73) Proprietor: **MONTEDIPE S.p.A.**
**31, Foro Buonaparte**
**Milan (IT)**

(72) Inventor: **Colombo, Fausto**
**60/A, Via Verdi**
**Merate (Como) (IT)**
Inventor: **Alberti, de Giordano**
**4, Largo Brianzoni**
**Besnate (Varese) (IT)**
Inventor: **Padovan, Mario**
**1, Via Villa Mirabello**
**Milano (IT)**
Inventor: **Paparatto, Guiseppe**
**8, Via Buozzi**
**Cinisello Balsamo (Milano) (IT)**
Inventor: **Contessa, Socrate**
**35, Via Repubblica**
**Senago (Milano) (IT)**

(74) Representative: **Barz, Peter, Dr. et al**
**Patentanwälte Dr. V. Schmied-Kowarzik Dipl.-Ing. G. Dannenberg Dr. P. Weinhold Dr. D. Gudel**
**Dipl.-Ing. S. Schubert Dr. P. Barz**
**Siegfriedstrasse 8**
**D-8000 München 40 (DE)**

Courier Press, Leamington Spa, England.

# 0 109 060

**Description**

Nowadays huge amounts of olefin cuts, from $C_4^-$ to $C_{12}^-$, linear or branched, are available throughout the world and they are widely employed for different purposes such as described, for instance, in Italian patent publications 24152 A/82, 24550 A/82 and 19292 A/83. Sometimes, however, because of contingent reasons, even outside the chemical field such as, for instance, transportation difficulties, it would be better to have still further possibilities of use. A promising use of said olefins would be their conversion into propylene and/or ethylene.

EP—A—37671 discloses a process for the acid catalyzed conversion of larger olefins to smaller olefins, wherein the larger olefins are contacted with a zeolitic compound, e.g. ZSM5 or ZSM11 zeolites, having a silica to alumina mole ratio of at least 12, the zeolitic compound being used as such or in a modified form.

However, endurance tests have shown that the excellent initial behavior of some catalysts such as, for instance, ZSM5 and ZSM11, disappears after some time; after a few weeks conversion and selectivity drop to poor levels.

An object of the invention is to provide a process for catalytically converting olefin cuts $C_4^-$—$C_{12}^-$ with high propylene (and, optionally, also ethylene) yields and with a long life time of the catalyst before substitution or regeneration.

In its most general form, the invention concerns a process for the conversion of linear butenes to propylene, which comprises contacting said butenes with a zeolitic compound, optionally in admixture with a binder, characterized in that said zeolitic compound is selected from silicalites, boralites, chromosilicates and those zeolites ZSM5 and ZSM11 in which the mole ratio $SiO_2/Al_2O_3$ is $\geq 350$ and that said conversion reaction is carried out at a temperature of from 500 to 600°C and at a space velocity of from 5 to 200 kg/h of butenes per kg of pure zeolitic compound (binder excluded).

The zeolitic compound is used as such or in a modified form.

The behavior of silicalites depends on the conversion pressure; for instance, if the silicalite is silicalite-1 and if the pressure is substantially atmospheric, the space velocity must be lower than 50 $h^{-1}$; if said compound is silicalite-1 and if the pressure is from 1.52 to 7.60 bar (1.5 to 7.5 absolute atmospheres) the space velocity must be in general above 50 $h^{-1}$.

Best results are obtained when said catalytic silicalite-1 is activated in the conversion reaction of the olefins into propylene, under said operative conditions. This initial (activating) run takes at least 8 and preferably 12 hours; the silicalite-1 is used as such or in a modified form and the modifying element is selected from Cr, Mg, Ca, Sr and Ba.

The modifying element can be incorporated into the catalyst by means of ion exchange or by any other method, for instance impregnation or co-precipitation during the synthesis of the zeolitic compound.

According to a particularly advantageous way for the preparation of a non-modified silicalite, the raw product coming from the zeolite synthesis is dried, for instance at 120°C, calcined (e.g. at 540°C for some hours), in order to remove all the residual organic templating agent, and then exchanged with an aqueous solution of HCl, $NH_4Cl$, $NH_4NO_3$ or an equivalent $H^+$ or $NH_4^+$ source. When an ammonium compound is used, it is necessary to heat, e.g. at 400°C, in order to obtain the acid form of the silicate. A survey of techniques alternative to ion exchange is given e.g. in U.S. patents 3,140,249; 3,140,251; 3,140,253 and in European patent publication 30796, 36707, 37168, 40463, 68754.

The zeolitic material, after calcination and conversion into the acid form, show a long endurance and a very high catalytic activity. These zeolitic compounds can be used as catalysts (as such or in a modified form) optionally in admixture with suitable amounts of binders, for instance $SiO_2$ or $Al_2O_3$. A list of other binders can be found e.g. in European patent publication 36707. The regeneration can be carried out in air for some hours, at 400—600°C. A steam regeneration is described in European patent publication 36704 and according to a further and very successful method, the catalyst can be regenerated by a hydrogen treatment.

As to the initial activation of the catalyst, some methods are described in European patent publications 34444 and 35830; in general it is advisable to activate the catalyst for some hours in air, at 450—750°C (preferably 540—700°C). Furthermore the conversion itself (of olefins to propylene) has an activating effect on the zeolitic catalyst. In other words, catalyst and reaction affect each other in a mutual, reciprocal and beneficial activating reaction.

Any process for the conversion of more or less heavy olefinic cuts into propylene will be indicated hereafter, as a "post-pyrolysis" process. When the feed of a post-pyrolysis process is a mixture of olefins having 4 C atoms,, there is a considerable problem to be solved, because the $C_4$ cuts always contain substantial amounts of paraffins, in general, also having 4 C atoms, which paraffins pass (at 400—600°C) the zeolitic bed without taking part in any reaction. Furthermore a small amount of $C_4^-$ paraffins is produced by the post-pyrolysis process itself. The conversion to $C_3H_6$ could be enhanced by a recycle of the non-reacted $C_4^-$ olefins or of the $C_4^-$ olefins formed during the reaction. In such a case, however, an increasing accumulation of n-butane and of isobutane would take place. This drawback could be avoided by a separation of paraffins from olefins before feeding the reactor but such a separation is rather difficult. Butenes and isobutane cannot be isolated by a simple distillation and it is usually necessary to carry out an extractive distillation (a complicated technique), which is particularly burdensome for the $C_4^-$ cuts coming from catalytic cracking, where butane and isobutane may account for even 50% of the whole. The problem,

2

# 0 109 060

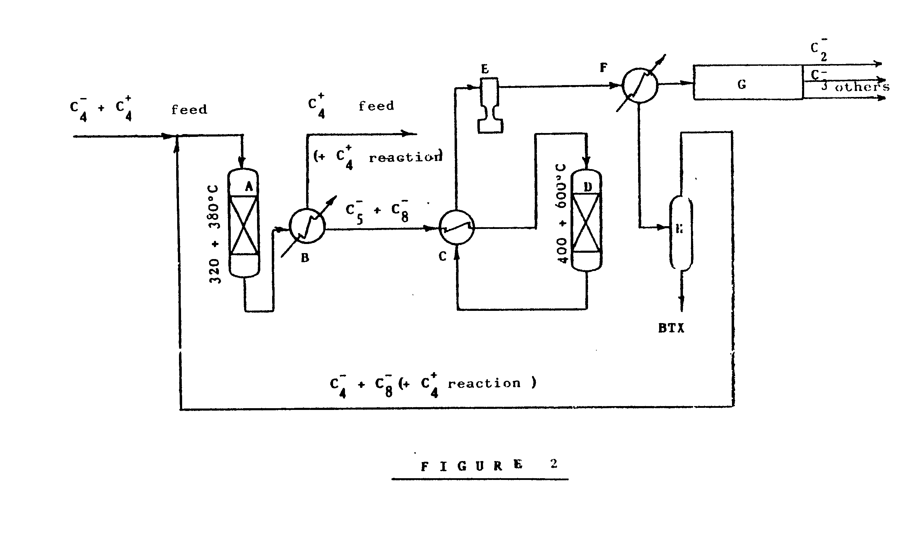

however, can be solved in a surprisingly easy way by the process according to the invention, when employing an integrated oligomerization step. In other words, a particualr embodiment of the invention (the feed being a paraffin-olefin mixture) comprises the following steps (reference is made also to figure 2):

a) preliminary oligomerization of a $(C_4^- + C_4^-)$ mixture at a temperature of from 320 to 380°C using a catalyst bed of zeolitic nature (see e.g. U.S. patent 4,150,062) to obtain a mixture of olefins having from 5 to 8 C atoms, with the $C_4^+$ paraffins remaining unconverted;

b) cooling and condensation of the oligomerization effluent in order to separate the $C_4^+$ paraffins as a gaseous phase, and conversion of the remaining $(C_5^- —C_8^-)$ mixture to propylene under typical "post-pyrolysis" operative conditions;

c) cooling of the effluent from the reactor for the conversion to propylene and compression of said effluent, preferably at 13—16 bar, whereby the hydrocarbons having 4 or more C atoms are condensed and the hydrocarbons having less than 4 C atoms are separated as a gaseous phase.

These hydrocarbons ($<C_4$) can be advantageously recycled to a conventional battery of distillation columns for thermal or catalytic cracking, in order to recover all the propylene contained therein. The small and possible amounts of aromatics (BTX) can be easily separated from the other $C_4$ hydrocarbons and recycled together with the final $(C_4^- —C_8^-)$ mixture, containing small amounts of butanes produced during the conversion reaction described under item (b) above.

In order to carry out the oligomerization, $C_4$ olefins, containing $C_4$ paraffins in any proportion, are initially brought into contact with a catalyst of zeolitic nature, for instance ZSM5 or ZSM11, in an acid or in a modified form, at 250—400°C (preferably 320—380°C) and at space velocities from 2 to 10 (preferably 4—8) kg/h of reactants per kg of pure zeolite (binder excluded). In other words, the olefins of the $(C_4^- + C_4^-)$ mixture are converted almost totally into a $(C_5^- —C_8^-)$ olefinic mixture, while butane and isobutane do not react. The separation of the butanes can thus be carried out very easily by simple cooling with water at room temperature. The olefinic $(C_5^- —C_8^-)$ mixture liquifies while the butanes are separated as a gaseous phase, said $(C_5^- —C_8^-)$ mixtures being optimal raw-materials for the production of propylene.

Depending on the operative conditions of the synthesis of the silicalite, as e.g. the dilution of the starting solutions, the resulting crystallites may have a widely variable size. The crystallites to be employed in the process according to the invention should have, in general, rather small sizes, such small sizes being obtainable, for instance, following the teachings of U.S. patent 3,926,782. The following examples are given merely for illustration purposes and do not in any way limit the scope of the invention.

Operative Conditions Common to all Examples

As to the method of preparation of the catalysts see:

— for silicalite-1: U.S. patent 4,061,724;

— for boralite: Taramasso et al.: "Molecular Sieve Borosilicates", Proc. 5th Int. Conf. on Zeolites, Naples 1980, pages 40—48 (Heyden and Son Ltd. London 1980); the boralite used in the examples has a ratio $SiO_2:B_2O_3$ of 7 (by moles); a more recent method for obtaining boro-silicates is described in European patent publication 77946;

— for chromosilicates: Italian patent publication 22568 A/82, in the name of the Applicant; the chromosilicate used in the examples has a ratio $SiO_2:Cr_2O_3$ of 38 (by moles).

As to zeolites ZSM5 showing a very high $SiO_2:Al_2O_3$ ratio, not exemplified, see Italian patent publication 21699 A/83, in the name of the Applicant. In the absence of different indications, all the catalysts were activated 2 hours at 540°C before being used.

Examples 1—6

3 g of zeolitic catalyst, in admixture with 0.9 g of $SiO_2$ (as a binder), were loaded into a microreactor which was continuously fed, at a pressure slightly above atmospheric pressure with a 50/50 mixture of butene-2-trans and butene-2-cis. Operative conditions and results are given in Table 1.

Example 7

Example 4 was repeated using a silicalite-1 impregnated with a chromium salt, thus obtaining slightly better results.

Examples 8—11

3 g of silicalite-1, without binder, were loaded into a microreactor which was continuously fed with 60 kg/h of the olefins of example 1 per kg of silicalite, at high pressures (6.08 bar for example 8 and 9 and 8.1 bar for example 10 and 11). The detailed operative conditions and the results are given in Table 2. The results show clearly that a slight increase in pressure, corresponding approximately to the pressure of the olefins in the industrial manufacturing plants, allows the same conversions and selectiveness, but at a much higher velocity. In other words, it is surprising and was quite unexpected that a suitable increase of the pressure increases very much the productivity of the silicate and therefore the output of the industrial plants.

Example 12

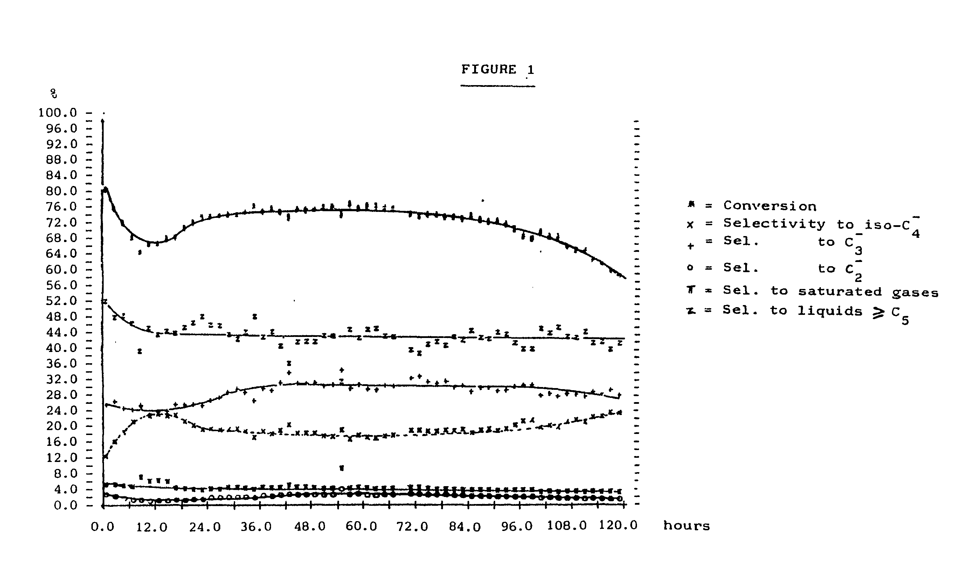

50 parts by weight of silicalite-1 were admixed with 50 parts of $Al_2O_3$ (binder) and the mixture was

**0 109 060**

loaded into a microreactor which was continuously fed at atmospheric pressure, with a 50/50 mixture of butene-2-trans and butene-2-cis, at 550°C and at a space velocity of 20 kg/h of olefins per kg of silicalite (binder excluded). The run was very long (120 h) and the results, hour by hour, were continuously monitored and plotted in figure 1. It is worthwhile to note that the initial decrease of conversion is reversed after a few hours; thus the conversion of the olefins itself is likely to be a stimulating activation for the catalyst. In other words, catalyst and reaction effect each other by a mutual and beneficial activating action.

Example 13

Example 12 was repeated by using a mixture 65% b.w. silicate-1+35% b.w. $Al_2O_3$ and by raising the space velocity to 67 $h^{-1}$. In this case, the conversion initially decreased and then the phenomenon was reversed.

4

TABLE 1

| Example | 1 | 2 | 3 | 4 | 5 | 6 |
|---|---|---|---|---|---|---|
| **Operative conditions:** | | | | | | |
| Catalyst | Boralite | See Ex. 1 | Silicalite—1 | See Ex. 3 | Chromosilicate | See Ex. 5 |
| $SiO_2/Al_2O_3$ (moles) | (b) | ,, | ∞ | ,, | (d) | ,, |
| Amount of catalyst | 3 grams (c) | ,, | See Ex. 1 | ,, | See 3x. 1 | ,, |
| Temp. (°C) | 500 | ,, | ,, | ,, | ,, | ,, |
| Space velocity (a) | 6 | ,, | ,, | ,, | ,, | ,, |
| Data survey after: | 1 h | 5 h | 1 h | 7 h | 1 h | 6 h |
| **Results (% b.w.)** | | | | | | |
| Conversion | 45.95 | 29.01 | 81.99 | 77.06 | 29.75 | 12.25 |
| Selectivity to iso-$C_4^-$ | 29.79 | 40.40 | 15.26 | 19.47 | 34.07 | 40.46 |
| Selectivity to $C_3^-$ | 28.36 | 24.90 | 44.79 | 40.39 | 29.73 | 23.65 |
| Yield (iso $C_4^-$ + $C_3^-$) | 26.72 | 18.94 | 49.23 | 45.13 | 19.88 | 7.85 |
| Sel. to compounds $\geqslant C_5$ | 38.76 | 31.73 | 28.15 | 31.75 | 32.29 | 30.82 |
| Sel. to saturated compounds $\leqslant C_4$ | 2.51 | 2.55 | 6.85 | 5.44 | 3.46 | 4.77 |
| Selectivity to $C_2^-$ | 0.57 | 0.43 | 4.95 | 2.95 | 0.45 | 0.31 |

(a) WHSV (Weight Hourly Space Velocity) namely Kg /h of olefine per Kg. of pure catalyst (binder excluded);

(b) $SiO_2 : B_2O_3 = 7$ (by moles);

(c) + 0.9 g of $SiO_2$ as binder;

(d) $SiO_2 : Cr_2O_3 = 38$ (by moles).

TABLE 2

| Example | 8 | 9 | 10 | 11 |
|---|---|---|---|---|
| Operative conditions : | ( * ) | ( * ) | ( ** ) | ( ** ) |
| Catalyst | Silicalite–1 | See Ex. 8 | See Ex. 8 | See Ex. 10 |
| $SiO_2/Al_2O_3$ (moles) | ∞ | ,, | ,, | ,, |
| Amount of catalyst | 3 g | ,, | ,, | ,, |
| Temp. (°C) | 570 | ,, | 580 | ,, |
| Space velocity ($h^{-1}$) | 60 | ,, | 60 | ,, |
| Data survey after : | 1 h | 8 h | 1 h | 8 h |
| Results (% b.w.) | | | | |
| Conversion | 82.24 | 73.80 | 79.31 | 67.32 |
| Selectivity to $C_3$ | 31.98 | 39.89 | 39.66 | 26.00 |
| Yield ($C_3^-$) | 26.30 | 29.44 | 31.45 | 17.50 |
| Sel. to $C_2^-$ | 3.89 | 3.19 | 4.21 | 3.20 |
| Sel. to saturated compounds $<C_4$ | 13.35 | 10.62 | 14.14 | 8.40 |
| Sel. to compounds $\geqslant C_5$ | 36.02 | 25.73 | 27.80 | 45.60 |
| Sel. to isobutene | 14.76 | 20.58 | 14.19 | 16.50 |

( * ) Pressure = 6 absolute atmospheres (6.08 bar).

( ** ) Pressure = 9 absolute atmospheres (9.12 bar).

# 0 109 060

**Claims**

1. A process for the conversion of linear butenes to propylene, which comprises contacting said butenes with a zeolitic compound, optionally in admixture with a binder, characterized in that said zeolitic compound is selected from silicalites, boralites, chromosilicates and those zeolites ZSM5 and ZSM11 in which the mole ratio $SiO_2/Al_2O_3$ is $\geq$350 and that said conversion reaction is carried out at a temperature of from 500 to 600°C and at a space velocity of from 5 to 200 kg/h of butenes per kg of pure zeolitic compound (binder excluded).

2. The process of claim 1 wherein said zeolitic compound is silicalite-1.

3. The process of claim 2 wherein the space velocity is from 5 to 50 $h^{-1}$, when the reaction pressure is substantially atmospheric, and is from 50 to 200 $h^{-1}$, when the reaction pressure is from 1.5 to 7.5 bar.

4. The process of claim 2 or 3 wherein the silicalite-1 is activated by the conversion reaction of said butenes to propylene under the reaction conditions of claim 1, the initial (activating) run being carried out at least 8 and, preferably, 12 hours.

5. The process of any one of claims 2 to 4 wherein said silicalite-1 is in a non-modified form.

6. The process of any one of claims 2 to 4 wherein said silicalite-1 is in a modified form, the modifying element being selected from Cr, Mg, Ca, Sr or Ba.

7. The process of any one of claims 1 to 6 wherein said binder is $SiO_2$.

**Patentansprüche**

1. Verfahren zur Umwandlung von linearen Butenen in Propylen, bei dem die Butene mit einer zeolithischen Verbindung, gegebenenfalls im Gemisch mit einem Bindemittel, in Kontakt gebracht werden, dadurch gekennzeichnet, daß die zeolithische Verbindung ausgewählt ist unter Silicaliten, Boraliten, Chromosilicaten und Zeolithen ZSM5 und ZSM11, bei denen das Molverhältnis $SiO_2/Al_2O_3$ $\geq$350 ist, und daß die Umwandlungsreaktion bei einer Temperatur von 500 bis 600 °C und einer Raumgeschwindigkeit von 5 bis 200 kg/h Butenen pro kg reiner Zeolithischer Verbindung (Bindemittel ausgenommen) durchgeführt wird.

2. Verfahren nach Anspruch 1, worin die zeolithische Verbindung Silicalit-1 ist.

3. Verfahren nach Anspruch 2, worin die Raumgeschwindigkeit 5 bis 50 $h^{-1}$ beträgt, wenn der Reaktionsdruck im wesentlichen Atmosphärendruck ist, und 50 bis 200 $h^{-1}$ beträgt, wenn der Reaktionsdruck 1,5 bis 7,5 bar ist.

4. Verfahren nach Anspruch 2 oder 3, worin der Silicalit-1 durch die Umwandlungsreaktion der Butene im Propylene unter den Reaktionsbedingungen von Anspruch 1 aktiviert wird, wobei der anfängliche (aktivierende) Durchgang mindestens 8 und vorzugsweise 12 Stunden durchgeführt wird.

5. Verfahren nach irgendeinem der Ansprüche 2 bis 4, worin der Silicalit-1 in nicht-modifizierter Form vorliegt.

6. Verfahren nach irgendeinem der Ansprüche 2 bis 4, worin der Silicalit-1 in modifizierter form vorliegt, wobei das modifizierende Element ausgewählt ist unter Cr, Mg, Ca, Sr oder Ba.

7. Verfahren nach irgendeinem der Ansprüche 1 bis 6, worin das Bindemittel $SiO_2$ ist.

**Revendications**

1. Un procédé de conversion de butènes linéaires en propylène, qui comprend le contact des butènes avec un composé zéolitique, éventuellement associé à un liant, caractérisé en ce que le dit composé zéolitique est choisi parmi les silicalites, les boralites, les chromosilicates et les zéolites ZSM5 et ZSM11 dans lesquelles le rapport molaire $SiO_2/Al_2O_3$ est supérieur ou égal à 350 et en ce que la dite réaction de conversion est mise en oeuvre à une température comprise entre 500°C et 600°C et à une vitesse spatiale comprise entre 5 et 200 kg/h de butènes par kilogramme de composé zéolitique pur (liant exclus).

2. Le procédé selon la revendication 1 dans lequel ledit composé zéolitique est la silicalite-1.

3. Le procédé selon la revendication 2 dans lequel la vitesse spatiale est comprise entre 5 et 50 $h^{-1}$, lorsque la pression réactionnelle est de l'ordre de la pression atmosphérique et est comprise entre 50 et 200 $h^{-1}$, lorsque la pression réactionnelle est comprise entre 1,5 et 7,5 bars.

4. Le procédé selon la revendication 2 ou 3 dans lequel, dans les conditions de réaction de la revendication 1, la silicalite-1 est activée par la réaction de conversion des dits butènes en propylène, la procédure initiale (activation) étant effectuée en au moins 8 heures, de préférence 12 heures.

5. Le procédé selon la revendication 1 dans lequel la silicalite-1 est sous une forme non modifiée.

6. Le procédé selon une quelconque des revendications 2 à 4 dans lequel ledit silicalite-1 est sous une forme modifiée, l'élément modificateur consistant en Cr, Mg, Ca, Sr ou Ba.

7. Le procédé selon une quelconque des revendications 1 à 6 dans lequel ledit liant est formé de $SiO_2$.

7

FIGURE 1

FIGURE 2